# EUROPÄISCHE PATENTANMELDUNG

(11) **EP 1 867 624 A1**
(43) Veröffentlichungstag der Anmeldung: **19.12.2007**
(21) Anmeldenummer: 07011026.7
(22) Anmeldetag: 05.06.2007
(51) Int. Cl.: C07C 17/12, C07C 25/18

(54) **Verfahren zur Herstellung von 2-Brom-4,4'-Diakyl-biphenyl**

(30) Priorität: 14.06.2006 DE 102006027491
(71) Anmelder: Saltigo GmbH, 40764 Langenfeld (DE)
(72) Erfinder: Rampf, Florian, Dr., 68220 Hegenheim (FR)
(74) Vertreter: Pettrich, Klaus-Günter

(57) **Zusammenfassung**

Verfahren zur Herstellung von 2-Brom-4,4'-dialkylbiphenyl, dadurch gekennzeichnet, dass man 4,4'-Dialkyl-biphenyl in Gegenwart von katalytischen Mengen Iod und gegebenenfalls in einem halogenierten Kohlenwasserstoff als Lösungsmittel mit Brom umsetzt.

## Beschreibung

Die vorliegende Erfindung betrifft ein Verfahren zur Herstellung von 2-Brom-4,4'-dialkylbiphenyl, insbesondere 2-Brom-4,4'-di-tert.-butylphenyl. Diese Bromverbindungen spielen eine wichtige Rolle bei der Synthese von Liganden für C-C-Kopplungen, z.B. lässt sich daraus das Grundgerüst von Liganden für die sogenannte Buchwald-Reaktion aufbauen.

J.Org. Chem., 1979, 44 (17), 3037-3041 beschreibt die Bromierung von 4,4'-Di-tert.-butylbiphenyl mit Brom in Gegenwart von Eisen als Katalysator in einer Ausbeute von 73 %. Hierbei wird zunächst Biphenyl zu 4,4'-Di-tert.-butylbiphenyl umgesetzt. Dann wird bromiert, wobei die tert.-Butylgruppe als dirigierend wirkende Schutzgruppe dient. Anschließend wird die Schutzgruppe abgespalten und das Produkt somit freigesetzt. Dabei zeigt sich, dass diese Synthese unter. Verwendung von Eisen als Katalysator nicht zur gewünschten Selektivität der Bromierung und damit Ausbeute führt. Neben dem gewünschten monobromierten Produkt entstehen isomere monobromierte und dibromierte Produkte, sowie Produkte, die eine oder beide tert.-Butylgruppen verloren haben. Entsprechend schwierig ist es, das gewünschte Produkt 2-Brom-4,4'-di-tert.-butylbiphenyl aus der Reaktionsmischung zu isolieren. Darüber hinaus wird als Lösungsmittel der Bromierung der in der technischen Umsetzung problematische Tetrachlorkohlenstoff eingesetzt.

Der vorliegenden Erfindung lag daher die Aufgabe zugrunde, ein Verfahren zur Herstellung von 2-Brom-4,4'-dialkylbiphenyl durch Bromierung der entsprechenden 4,4'-Dialkyl-biphenyl-Verbindung in fast vollständiger Selektivität und sehr guter Ausbeute zu erhalten. Es wurde nun überraschenderweise festgestellt, dass diese Reaktion in fast vollständiger Selektivität und sehr guter Ausbeute erfolgt, wenn die Bromierung in Gegenwart katalytischer Mengen an Iod (I₂) durchgeführt wird.

Gegenstand der Erfindung ist daher ein Verfahren zur Herstellung von 2-Brom-4,4'-dialkylbiphenyl, bei dem man 4,4'-Dialkylbiphenyl in Gegenwart von katalytischen Mengen an Iod und gegebenenfalls halogenierten Kohlenwasserstoff als Lösungsmittel mit Brom umsetzt.

Als 4,4'-Alkylbiphenyl-Verbindungen kommen dabei die entsprechenden Methyl-, Ethyl-, Propylsowie Butyl-Verbindungen in Frage. Bevorzugt ist dabei 4,4'-Di-tert.-butylbiphenyl, welches durch die Bromierung zum gewünschten Produkt 2-Brom-4,4'-di-tert.-butylbiphenyl führt. Das Jod liegt dabei als elementares Jod in einer katalytischen Menge, z.B. in einer Menge von 0,1 bis 10 Mol-%, bezogen auf den zu bromierenden Aromaten vor. Bevorzugt beträgt die eingesetzte Jodmenge 1 bis 5 Mol-%, bezogen auf den zu bromierenden Aromaten.

Die Reaktion kann ohne Lösungsmittel, bevorzugt in einem nichtwässrigen Lösungsmittel, wie einem halogenierten Kohlenwasserstoff durchgeführt werden. Als halogenierte Kohlenwasserstoffe können dabei alle flüssigen Kohlenwasserstoffverbindungen aus der Gruppe Methylenchlorid, 1,2-Dichlorethan, Chloroform, Chlorbenzol, Dichlorbenzol verwendet werden. Bevorzugt setzt man Methylenchlorid als Lösungsmittel ein.

Üblicherweise erfolgt die Umsetzung bei einer Temperatur im Bereich von -5 bis 40°C. Die bevorzugte Reaktionstemperatur ist 5°C.

Die Reaktionszeit für die erfindungsgemäße Umsetzung liegt im Bereich zwischen einer und 10 Stunden. Bevorzugt beträgt die Reaktionszeit ein bis zwei Stunden. Im erfindungsgemäßen Verfahren ist es überraschenderweise möglich, die genannten Brombiphenyl-Verbindungen in hoher Selektivität und Ausbeute zu erhalten. Üblicherweise liegt die Selektivität bei mehr als 98% und die Ausbeute bei mehr als 95%.

Das nachfolgende Beispiel soll die Erfindung näher erläutern ohne sie jedoch in ihrem Umfang einzuschränken.

### Beispiel 1

1 g 4,4'-Di-tert.-butyl-biphenyl wurden in 10 g Dichlormethan gelöst und mit 30 mg Iod versetzt. Anschließend wurde auf 5°C gekühlt. Über eine Zeitspanne von 1 h wurden 650 mg Brom zugegeben. Dann wurde 1 h bei Raumtemperatur nachgerührt. Zur Aufarbeitung wurden 3 ml einer 10 %igen Lösung von NaHSO₃ zugegeben, Phasen getrennt, neutral gewaschen, getrocknet und einrotiert. Es wurden 1,28 g 2-Brom-4,4'-di-tert.-butyl-biphenyl erhalten (98,6 % d.Th.).

## Patentansprüche

1. Verfahren zur Herstellung von 2-Brom-4,4'-dialkyl-biphenyl, **dadurch gekennzeichnet, dass** man 4,4'-Dialkyl-biphenyl in Gegenwart von katalytischen Mengen Iod und gegebenenfalls in einen halogenierten Kohlenwasserstoff als Lösungsmittel mit Brom umsetzt.

2. Verfahren nach Anspruch 1, **dadurch gekennzeichnet, dass** das 2-Brom-4,4'-dialkylbiphenyl 2-Brom-4,4'-di-tert.-butylbiphenyl ist.

3. Verfahren nach einem der Ansprüche 1 bis 2, **dadurch gekennzeichnet, dass** das Iod in einer Menge von 0,1-10 Mol-%, bezogen auf das 4,4'-Dialkyl-biphenyl, eingesetzt wird.

4. Verfahren nach einem der vorherigen Ansprüche, **dadurch gekennzeichnet, dass** der halogenierte Kohlenwasserstoff Methylenchlorid ist.

5. Verfahren nach einem der vorherigen Ansprüche, **dadurch gekennzeichnet, dass** man die Umsetzung bei einer Temperatur in Bereich von 0-40°C durchführt.

6. Verfahren nach einem der vorherigen Ansprüche, **dadurch gekennzeichnet, dass** die Reaktionszeit zwischen einer und 10 h beträgt.
